# EUROPÄISCHE PATENTSCHRIFT

(11) **EP 3 015 081 B1**
(45) Veröffentlichungstag und Bekanntmachung des Hinweises auf die Patenterteilung: **02.10.2019**
(21) Anmeldenummer: 15190736.7
(22) Anmeldetag: 21.10.2015
(51) Int. Cl.: A61B 17/29, A61B 17/00, A61B 34/00

(54) **CHIRURGISCHES INSTRUMENT MIT EINER MANUELLEN STEUERVORRICHTUNG**
SURGICAL INSTRUMENT WITH A MANUAL CONTROL DEVICE
INSTRUMENT CHIRURGICAL DOTE D'UN DISPOSITIF DE COMMANDE MANUEL

(30) Priorität: 27.10.2014 DE 102014115600
(43) Veröffentlichungstag der Anmeldung: 04.05.2016
(73) Patentinhaber: Karl Storz SE & Co. KG, 78532 Tuttlingen (DE)
(72) Erfinder: Professor Dr. Omid, Abri, 14129 Berlin (DE); Schrader, Stephan, 14532 Kleinmachnow (DE); Forster, Jonas, 13595 Berlin (DE)
(74) Vertreter: Witte, Weller & Partner Patentanwälte mbB

(56) Entgegenhaltungen:
- WO-A1-99/18863
- WO-A1-2013/018912
- WO-A2-2012/127404
- GB-A- 2 302 655
- US-A1- 2013 331 826

## Beschreibung

Die vorliegende Erfindung betrifft ein chirurgisches Instrument mit einer manuellen Steuervorrichtung für eine Betätigung mit der Hand, wobei die Steuervorrichtung zwischen einer geschlossenen Position und einer geöffneten Position verlagert werden kann. Die Verlagerung erfolgt mittels manueller Einwirkung auf die Steuervorrichtung durch die Hand eines Benutzers, indem dieser die Hand öffnet und schließt.

Es gibt eine Vielzahl von Werkzeugen und Instrumenten, die ein Benutzer manuell mit seiner Hand betätigt. Einige dieser Instrumente erfordern bei der Betätigung eine besondere Ergonomie, z.B. weil die Betätigung des Instruments besonders feinfühlig möglich sein muss, eine häufige wiederholte Betätigung des Instruments erforderlich ist oder bestimmte Betätigungspositionen des Instruments über längere Zeit konstant gehalten werden müssen.

Zu diesen Instrumenten zählen auch chirurgische Instrumente, insbesondere minimalinvasive chirurgische Instrumente. Diese haben am proximalen Ende mindestens einen Griff, der es einem Benutzer, insbesondere einem Chirurgen oder einem Assistenten, erlaubt, das Instrument zu halten und zu betätigen. Solche Instrumente müssen ergonomisch greifbar und ergonomisch betätigbar sein, dies auch für beliebige Handgrößen. Schließlich muss es dem Benutzer auch möglich sein, wiederholt auf bequeme Weise und in verschiedenen Lagen der Steuervorrichtung eine ausreichende Kraft auf die Steuervorrichtung ausüben zu können.

Eine Vielzahl der Griffe für solche Instrumente sind im Wesentlichen pistolenförmig. Diese Griffform kann in einem breiten Winkelbereich gut gegriffen werden, wobei auch in unterschiedlichen Lagen und bei ungünstigen Haltewinkeln gut gearbeitet werden kann. Oft verfügen diese Griffe über Fingerschlaufen, mit denen ausreichend feinfühlig gearbeitet werden kann.

In diesem Zusammenhang sei lediglich beispielhaft auf die Steuervorrichtungen hingewiesen, die in US 6,299,624 B1, US 2007/0005002 A1 und US 2008/0154246 A1 gezeigt sind. Außerdem sei auf US 5,976,121 hingewiesen, wo eine besonders ergonomische Steuervorrichtung gezeigt ist.

US 2013/0331826 A1 zeigt eine Bedienvorrichtung für ein chirurgisches Instrument. Die Bedienung des Instruments erfolgt u.a. durch einen Daumenhebel und einen Fingerhebel, die über ein Gestänge mit einem Drehelement verbunden sind. Das Drehelement ist mit einem Aktuator des Instruments verbunden. Durch ein Öffnen und Schließen des Daumenhebels und des Fingerhebels dreht sich das Drehelement und der Aktuator wird betätigt.

WO 2013/018912 A1 zeigt eine Fernsteuerung für ein chirurgisches Instrument, welches über einen robotischen Manipulator betätigt wird. Bei der Benutzung der Fernsteuerung wird ermittelt, welches Objekt mittels der Fernsteuerung bedient werden soll. Dann wird eine Verlagerung oder Betätigung der Fernsteuerung ermittelt, die dann über den Manipulator in eine Betätigung des chirurgischen Instruments umgesetzt wird.

WO 2012/127404 A2 zeigt eine Vorrichtung, die verschiedene Bewegungen der menschlichen Hand erfassen kann, einschließlich eines Drehens und eines Kippens der Hand.

WO 99/18863 A1 zeigt ein Instrument für endoskopische Anwendungen mit einer proximalen Bedieneinheit und einem distalen Funktionselement, dessen einzelne Teile gemeinsam oder unabhängig voneinander bewegbar sind. Die Bedieneinheit kann mit einer Hand umgriffen werden und mittels zweier schwenkbarer und/oder kippbarer Elemente bedient werden.

GB 2 302 655 A offenbart ein Handstück für laparoskopische Instrumente.

Trotz der großen Anzahl an manuellen Steuervorrichtungen besteht auch weiterhin ein Bedarf an Steuervorrichtungen, die dem Benutzer eine bequeme und intuitive Bedienung auch über einen längeren Zeitraum ermöglichen.

Es ist eine Aufgabe der vorliegenden Erfindung, ein verbessertes chirurgisches Instrument mit einer manuellen Steuervorrichtung für eine Betätigung mit der Hand aufzuzeigen, die eine bequeme und ergonomische Betätigung, insbesondere durch ein Öffnen und Schließen der Hand, ermöglicht.

Bei einem ersten Aspekt wird die Aufgabe gelöst durch ein chirurgisches Instrument mit einer manuellen Steuervorrichtung für eine Betätigung mit einer Hand,
wobei die Steuervorrichtung zwischen einer geschlossenen Position und einer geöffneten Position verlagert werden kann und die Steuervorrichtung aufweist:
- einen Fingerhebel mit einem ersten Anlenkpunkt und einem zweiten Anlenkpunkt, wobei der Fingerhebel einen Fingerauflageabschnitt aufweist, der ausgehend vom ersten Anlenkpunkt als Fortsatz einer ersten Erstreckung des Fingerhebels zwischen dem zweiten Anlenkpunkt und dem ersten Anlenkpunkt ausgebildet ist,
- ein Zentralelement mit einem dritten Anlenkpunkt und einem vierten Anlenkpunkt,
- einen Verbindungshebel, der am ersten Anlenkpunkt und am dritten Anlenkpunkt verschwenkbar angeordnet ist, so dass der Verbindungshebel relativ zum Zentralelement und relativ zum Fingerhebel verschwenkbar ist und dass der erste Anlenkpunkt entlang einer ersten gekrümmten Bahn um den dritten Anlenkpunkt herum geführt verlagerbar ist,
- einen Daumenhebel, der am zweiten Anlenkpunkt und am vierten Anlenkpunkt verschwenkbar angeordnet ist, so dass der Daumenhebel relativ zum Zentralelement und relativ zum Fingerhebel verschwenkbar ist und dass der zweite Anlenkpunkt entlang einer zweiten gekrümmten Bahn um den vierten Anlenkpunkt herum geführt verlagerbar ist.

Im Rahmen der zu erzielenden Verbesserung wurde erkannt, dass es ein Problem bei bekannten manuellen Steuervorrichtungen ist, dass diese die tatsächliche Bewegung der Hand eines Benutzers beim Öffnen und Schließen der Hand nicht ausreichend berücksichtigen. Dies führt dazu, dass bekannte manuelle Steuervorrichtungen zwar bei Betrachtung der vorgeschlagenen Technik als ergonomisch und bequem bedienbar erscheinen, dies in der Praxis, gerade bei länger andauernden Prozeduren, vom Benutzer nicht so empfunden wird.

In diesem Zusammenhang wurde ferner erkannt, dass zumindest ein Teil der Problematik darin zu liegen scheint, dass sich Daumen und Finger einer Hand beim Öffnen nicht wie bei einem Scharnier genau auf einer Kreisbahn bewegen. Vielmehr wurde erkannt, dass sich Daumen und Finger beim Öffnen der Hand zunächst fast geradlinig voneinander entfernen. Der Daumen schwenkt dann recht schnell auf eine gekrümmte Bahn ein, insbesondere eine Kreisbahn, während die Finger sich weiterhin auf einer zumindest in etwa geradlinigen Bahn fortbewegen. Erst nachdem der Öffnungsprozess der Hand schon deutlich fortgeschritten ist, schwenken auch die Finger auf eine gekrümmte Bahn ein, insbesondere auf eine Kreisbahn.

Bei der hier offenbarten manuellen Steuervorrichtung wurde zudem berücksichtigt, dass eine solche Steuervorrichtung auch kostengünstig zu realisieren sein soll. Daher wurde gezielt nach einer Lösung gesucht, die die natürliche Öffnungsbewegung der Hand besser nachempfindet und dennoch einen möglichst einfachen mechanischen Aufbau hat. Ein mechanisch einfacher Aufbau bietet dabei sowohl Vorteile im Hinblick auf die Zuverlässigkeit der Steuervorrichtung als auch im Hinblick auf eine kostengünstige Fertigung der Steuervorrichtung.

Eine Besonderheit der offenbarten manuellen Steuervorrichtung ist, dass sich die verschiedenen Hebel, also Fingerhebel, Verbindungshebel und Daumenhebel, zwar um Anlenkpunkte verschwenken, sich also eigentlich auf einer kreisförmigen Bahn bewegen, sich aber gleichzeitig zumindest einer der Anlenkpunkte, insbesondere genau zwei oder mindestens zwei der Anlenkpunkte, verlagert, so dass das distale Ende des Fingerhebels zumindest während eines Abschnitts des Öffnungsvorgangs sich entlang eines zumindest ungefähr linearen Wegs verlagert.

Bei einigen bevorzugten Ausgestaltungen wird die Bewegung mindestens eines der genannten Hebel mechanisch oder elektrisch erfasst und zum Betätigen, insbesondere für ein Öffnen und Schließen, des chirurgischen Instruments verwendet. Bei einigen bevorzugten Ausgestaltungen bewegen sich bei einer Betrachtung entlang der Längsrichtung der erste Anlenkpunkt und der zweite Anlenkpunkt beim Öffnen der Steuervorrichtung in Richtung der distalen Seite und beim Schließen in Richtung der proximalen Seite. Bei einigen bevorzugten Ausgestaltungen hat der Fingerauflageabschnitt eine Breite senkrecht zur Längsrichtung und, insbesondere, auch senkrecht zur Höhenrichtung von mindestens 5 mm, bevorzugt mindestens 10 mm, besonders bevorzugt von mindestens 20 mm und insbesondere von mindestens 30 mm.

Für eine vereinfachte Orientierung sollen hier und im Folgenden die Begriffe distal und proximal wie folgt verwendet werden. Als proximal soll der Teil der Steuervorrichtung verstanden werden, der dem Mittelpunkt einer Hand des Benutzers am nächsten ist, wenn dieser seine Hand auf oder in die Steuervorrichtung gelegt hat. Die distale Seite der Steuervorrichtung liegt in Längserstreckung der Steuervorrichtung der proximalen Seite gegenüber. Hier befinden sich die Fingerspitzen des Benutzers, wenn er seine Hand auf oder in die Steuervorrichtung gelegt hat. Die Längsrichtung von proximal nach distal kann dabei insbesondere als eine Gerade verstanden werden, die bei geschlossener Steuervorrichtung durch den zweiten und den dritten Anlenkpunkt führt. Diese Orientierungshilfen dienen lediglich einem besseren Verständnis der Erfindung.

Wie anhand von Ausführungsbeispielen später noch verdeutlicht wird, verlagert sich ein erstes distales Ende des Fingerhebels während eines Abschnitts des Öffnungsvorgangs, insbesondere zu Beginn des Öffnungsvorgangs, derart, dass die Position des ersten distalen Endes bezogen auf die Längsrichtung zumindest im Wesentlichen unverändert bleibt. Dies wird u.a. dadurch erreicht, dass während sich der Fingerhebel um den ersten Anlenkpunkt verschwenkt, der erste Anlenkpunkt gleichzeitig in Richtung der distalen Seite verlagert wird. So wird die Drehbewegung des Fingerhebels um den ersten Anlenkpunkt, die eigentlich eine Verlagerung des ersten distalen Endes in Richtung der proximalen Seite bewirken würde, durch die Verlagerung des ersten Anlenkpunkts in Richtung der distalen Seite zumindest teilweise kompensiert oder überkompensiert.

Um die Kompensation bzw. Überkompensation besser zu verstehen, sei nachfolgend angenommen, dass sich das erste distale Ende des Fingerhebels beim Öffnen um eine Strecke x entlang der Längsrichtung verlagert und entlang einer Strecke y entlang einer Höhenrichtung verlagert, die senkrecht zur Längsrichtung steht. Bei einigen bevorzugten Ausgestaltungen ist die Kompensation bzw. Überkompensation derart gewählt, dass die Strecke x, entweder in Richtung der distalen Seite oder in Richtung der proximalen Seite bei einem Öffnen der Steuervorrichtung um 45° ausgehend von der geschlossenen Position höchstens 0,25y, bevorzugt 0,2y, besonders bevorzugt 0,15y und insbesondere 0,1y beträgt. Zusätzlich oder alternativ ist die Kompensation bzw. Überkompensation derart gewählt, dass die Strecke x, entweder in Richtung der distalen Seite oder in Richtung der proximalen Seite bei einem Öffnen der Steuervorrichtung um 30° ausgehend von der geschlossenen Position höchstens 0,2y, bevorzugt 0,15y, besonders bevorzugt 0,1y und insbesondere 0,05y beträgt.

Bei einigen bevorzugten Ausgestaltungen ist die Steuervorrichtung derart ausgebildet, dass das Zentralelement bei einem Öffnen und Schließen der Steuervorrichtung relativ zu einem Handmittelpunkt ruht. Dies bedeutet, dass sich zwar die genannten Hebel verlagern, die Steuervorrichtung insgesamt aber bezogen auf den Handmittelpunkt ruht. Insbesondere wenn das Zentralelement mit einem chirurgischen Instrument gekoppelt ist, kann auf diese Weise erreicht werden, dass auch bei einem Öffnen und Schließen der Steuervorrichtung der Handmittelpunkt relativ zu dem chirurgischen Instrument ruht. Mit anderen Worten, ein Öffnen und Schließen der Steuervorrichtung bewirkt keine Verlagerung des Zentralelements oder des chirurgischen Instruments in Längsrichtung.

Bei einigen bevorzugten Ausgestaltungen beträgt eine Länge des Fingerauflageabschnitts zwischen 35% und 95% der Länge des Fingerhebels, bevorzugt zwischen 45% und 90%, besonders bevorzugt zwischen 55% und 85% und insbesondere zwischen 65% und 80%. Unter der Länge des Fingerauflageabschnitts soll dabei die Strecke vom ersten Anlenkpunkt zum distalen Ende des Fingerhebels verstanden werden. Unter der Länge des Fingerhebels soll die Strecke vom zweiten Anlenkpunkt über den ersten Anlenkpunkt bis zum distalen Ende des Fingerhebels verstanden werden.

Bei einigen bevorzugten Ausgestaltungen verlaufen die Schwenkachsen, also die Achsen, um die sich die Hebel an den Anlenkpunkten relativ zueinander oder relativ zum Zentralelement verschwenken, im Wesentlichen parallel zueinander. Zusätzlich oder alternativ verlaufen die Schwenkachsen quer zu einer auf die Steuervorrichtung aufliegenden Hand. Die Schwenkachsen können auch als parallel zu einer Breitenrichtung verstanden werden, die sowohl zur Längsrichtung als auch zur Höhenrichtung senkrecht steht.

Bei der ersten gekrümmten Bahn und/oder der zweiten gekrümmten Bahn handelt es sich insbesondere um eine Kreisbahn.

Damit ist die Aufgabe vollständig gelöst.

Bei einer bevorzugten Weiterbildung weist der Daumenhebel einen Daumenauflageabschnitt auf, der ausgehend vom vierten Anlenkpunkt als Fortsatz einer zweiten Erstreckung des Daumenhebels zwischen dem zweiten Anlenkpunkt und dem vierten Anlenkpunkt ausgebildet ist.

Bei dieser Weiterbildung wird auch der Daumen der Hand in die Bewegung beim Öffnen und Schließen der Steuervorrichtung einbezogen. Dies erlaubt eine besonders natürliche Betätigung mit der Hand. Bei einigen bevorzugten Ausgestaltungen beträgt eine Länge des Daumenauflageabschnitts zwischen 35% und 95% der Länge des Daumenhebels, bevorzugt zwischen 45% und 85%, besonders bevorzugt zwischen 55% und 75% und insbesondere zwischen 60% und 70%. Unter der Länge des Daumenauflageabschnitts soll dabei die Strecke vom vierten Anlenkpunkt zum distalen Ende des Daumenhebels verstanden werden. Unter der Länge des Daumenhebels soll die Strecke vom zweiten Anlenkpunkt über den vierten Anlenkpunkt bis zum distalen Ende des Daumenhebels verstanden werden.

Bei einer vorteilhaften Weiterbildung weist der Verbindungshebel einen Betätigungsabschnitt auf, der ausgehend vom dritten Anlenkpunkt als Fortsatz einer dritten Erstreckung des Verbindungshebels zwischen dem ersten Anlenkpunkt und dem dritten Anlenkpunkt ausgebildet ist.

Bei dieser Weiterbildung kann eine Kraft, die beim Öffnen und Schließen der Hand auf die Steuervorrichtung einwirkt, besonders einfach in eine Schubbewegung umgesetzt werden. Dabei können sowohl ein positiver Schub als auch ein negativer Schub, der auch als Zug bezeichnet wird, erzeugt werden.

Bei einer weiteren vorteilhaften Weiterbildung der Erfindung weist das Zentralelement einen fünften Anlenkpunkt auf und weist die Steuervorrichtung ein erstes Steuerelement auf, das einen sechsten Anlenkpunkt aufweist, am fünften Anlenkpunkt und am sechsten Anlenkpunkt verschwenkbar angeordnet ist und mit dem Verbindungshebel gekoppelt ist, so dass das erste Steuerelement relativ zum Zentralelement verschwenkbar ist und dass der sechste Anlenkpunkt entlang einer dritten gekrümmten Bahn um den fünften Anlenkpunkt herum geführt verlagerbar ist.

Diese Ausgestaltung ermöglicht eine gute Übertragung einer durch die manuelle Betätigung auf die Steuervorrichtung wirkenden Kraft. Da der fünfte Anlenkpunkt relativ zum Zentralelement fixiert ist, kann hier ein feststehender Teil eines Instruments angeordnet werden. Da sich der sechste Anlenkpunkt bei einer Betätigung der Steuervorrichtung verlagert, kann hier ein bewegliches Element des Instruments angekoppelt werden. Bei einer Betätigung der Steuervorrichtung bleibt die Steuervorrichtung insgesamt unbeweglich zum feststehenden Teil des Instruments, die Betätigung des Fingerhebels und ggf. des Daumenhebels erzeugt aber eine Bewegung des sechsten Anlenkpunkts und damit eine Verlagerung des beweglichen Teils des Instruments.

Bei einer weiteren vorteilhaften Ausgestaltung weist das Zentralelement einen siebten Anlenkpunkt auf und weist die Steuervorrichtung ein zweites Steuerelement auf, das einen achten Anlenkpunkt aufweist, am siebten Anlenkpunkt und am achten Anlenkpunkt verschwenkbar angeordnet ist und mit dem Verbindungshebel gekoppelt ist, so dass das zweite Steuerelement relativ zum Zentralelement verschwenkbar ist und dass der achte Anlenkpunkt entlang einer vierten gekrümmten Bahn um den siebten Anlenkpunkt herumgeführt verlagerbar ist.

Diese Ausgestaltung ermöglicht eine gute Übertragung einer durch die manuelle Betätigung auf die Steuervorrichtung wirkenden Kraft. Da der siebte Anlenkpunkt relativ zum Zentralelement fixiert ist, kann hier ein feststehender Teil eines Instruments angeordnet werden. Da sich der achte Anlenkpunkt bei einer Betätigung der Steuervorrichtung verlagert, kann hier ein bewegliches Element des Instruments angekoppelt werden. Bei einer Betätigung der Steuervorrichtung bleibt die Steuervorrichtung insgesamt unbeweglich zum feststehenden Teil des Instruments, die Betätigung des Fingerhebels und ggf. des Daumenhebels erzeugt aber eine Bewegung des achten Anlenkpunkts und damit eine Verlagerung des beweglichen Teils des Instruments.

Der fünfte Anlenkpunkt und der siebte Anlenkpunkt können von einander beabstandet sein, insbesondere entlang einer Höhenrichtung, die senkrecht zur Längsrichtung steht. Bei einigen bevorzugten Ausgestaltungen fallen der fünften Anlenkpunkt und der siebte Anlenkpunkt zusammen. Dann sind das erste und das zweite Steuerelement an einem gemeinsamen Anlenkpunkt angelenkt.

Bei einer weiteren vorteilhaften Weiterbildung sind der Verbindungshebel und das erste Steuerelement mit einer ersten gelenkigen Verbindung gekoppelt, insbesondere mit einem ersten Zwischenhebel.

Diese Ausgestaltung ermöglicht eine günstige Verlagerung des ersten Steuerelements.

Bei einer weiteren vorteilhaften Weiterbildung sind der Verbindungshebel und das zweite Steuerelement einer zweiten gelenkigen Verbindung gekoppelt, insbesondere mit einem zweiten Zwischenhebel.

Diese Ausgestaltung ermöglicht eine günstige Verlagerung des zweiten Steuerelements.

Bei einer weiteren vorteilhaften Weiterbildung ist der erste Anlenkpunkt um einen ersten Abstand vom zweiten Anlenkpunkt beabstandet, ist der dritte Anlenkpunkt um einen zweiten Abstand vom vierten Anlenkpunkt beabstandet und beträgt der erste Abstand zwischen 25% und 125% des zweiten Abstands, bevorzugt zwischen 35% und 100%, besonders bevorzugt zwischen 45% und 75% und insbesondere zwischen 50% und 60%.

Diese Abstandsverhältnisse haben sich bei praktischen Versuchen als besonders geeignet herausgestellt.

Bei einer weiteren vorteilhaften Weiterbildung ist der erste Anlenkpunkt um einen dritten Abstand vom dritten Anlenkpunkt beabstandet, ist der zweite Anlenkpunkt um einen vierten Abstand vom vierten Anlenkpunkt beabstandet und beträgt der vierte Abstand zwischen 35% und 150% des dritten Abstands, bevorzugt zwischen 45% und 125%, besonders bevorzugt zwischen 55% und 100% und insbesondere zwischen 65% und 85%.

Diese Abstandsverhältnisse haben sich bei praktischen Versuchen als besonders geeignet herausgestellt.

Bei einer weiteren vorteilhaften Weiterbildung ist der erste Anlenkpunkt um einen ersten Abstand vom zweiten Anlenkpunkt beabstandet, ist der zweite Anlenkpunkt um einen vierten Abstand vom vierten Anlenkpunkt beabstandet und beträgt der erste Abstand zwischen 40% und 150% des dritten Abstands, bevorzugt zwischen 45% und 125%, besonders bevorzugt zwischen 50% und 100% und insbesondere zwischen 60% und 80%.

Diese Abstandsverhältnisse haben sich bei praktischen Versuchen als besonders geeignet herausgestellt.

Bei einer weiteren vorteilhaften Weiterbildung weist die Steuervorrichtung ferner eine Schubstange auf, die gelenkig mit mindestens einem Element ausgewählt aus der Gruppe von Fingerhebel, Daumenhebel und Verbindungshebel gekoppelt ist.

Diese Ausgestaltung ermöglicht es auf einfache Weise, die Bewegung beim Öffnen und Schließen der Steuervorrichtung in eine zumindest näherungsweise lineare Schubbewegung umzusetzen. Dabei kann der Schub auch hier sowohl positiv als auch negativ sein. Bei einer bevorzugten Ausgestaltung wird die Schubstange gelenkig mit dem Daumenhebel gekoppelt, dabei insbesondere mit einem Abschnitt des Daumenhebels, der zwischen dem zweiten Anlenkpunkt und dem vierten Anlenkpunkt liegt.

Bei einer weiteren vorteilhaften Weiterbildung ist die Steuervorrichtung derart ausgebildet, dass sich bei einem Öffnen der Steuervorrichtung ein fünfter Abstand zwischen dem zweiten Anlenkpunkt und dem dritten Anlenkpunkt verringert.

Diese Ausgestaltung ermöglicht auf einfache Weise die zuvor beschriebene Kompensation bzw. Überkompensation bei der Verlagerung des Fingerhebels bzw. des ersten distalen Endes des Fingerhebels. In gleicher Weise vergrößert sich der fünfte Abstand beim Schließen der Steuervorrichtung.

Bei einer weiteren vorteilhaften Weiterbildung weist das Zentralelement eine Handballenauflage und/oder einen Adapter für eine Kopplung mit einem chirurgischen Instrument oder mit einem haptischen Eingabegerät auf.

Bei dieser Weiterbildung lässt sich die Steuervorrichtung besonders einfach mit einem Instrument koppeln. Zudem erlaubt die zusätzliche oder alternative Handballenauflage ein besonders angenehmes und entspanntes Greifen der Steuervorrichtung. Bei einigen bevorzugten Ausgestaltungen wird die manuelle Steuervorrichtung mit einem chirurgischen Instrument aus der Serie CLICKline der Firma Karl Storz gekoppelt.

Bei einer weiteren vorteilhaften Weiterbildung weist die Steuervorrichtung ferner einen Motor auf, der mit mindestens einem Element ausgewählt aus der Gruppe von Fingerhebel, Daumenhebel und Verbindungshebel zusammenwirkt, um eine haptische Rückmeldung und/oder eine motorische Unterstützung beim Öffnen und/oder Schließen der Steuervorrichtung zu geben.

Diese Ausgestaltung ermöglicht insbesondere eine Telemanipulation oder ein Arbeiten in einer virtuellen Testumgebung. Alternativ oder zusätzlich kann der Benutzer beim Öffnen und/oder Schließen der Steuervorrichtung oder beim Halten einer konstanten Position unterstützt werden.

Bei einer weiteren vorteilhaften Weiterbildung weist die Steuervorrichtung ferner eine Messeinrichtung auf, die dafür ausgebildet ist, einen Öffnungsgrad der Steuervorrichtung zu ermitteln, insbesondere einen Öffnungswinkel der Steuervorrichtung.

Diese Ausgestaltung ermöglicht ein vorteilhaftes Arbeiten mit der Steuervorrichtung, insbesondere dann, wenn die Steuervorrichtung nicht direkt mechanisch mit einem Aktuator des Instruments verbunden ist. Die Auflösung bei der Ermittlung des Öffnungsgrads kann je nach Bedarf gewählt werden. So wird bei einigen bevorzugten Ausgestaltungen lediglich zwischen den Zuständen offen und geschlossen unterschieden. Bei anderen bevorzugten Ausgestaltungen wird zwischen den Zuständen geschlossen, teilgeöffnet und geöffnet bzw. vollständig geöffnet unterschieden. Bei nochmals anderen Ausgestaltungen wird der Öffnungswinkel zumindest ungefähr ermittelt. Hierfür wird insbesondere ein Winkelmesser eingesetzt, der einen Winkel von mindestens einem der Hebel relativ zu einem der anderen Hebel oder dem Zentralelement ermittelt. Der ermittelte Wert des Öffnungsgrads der Steuervorrichtung, insbesondere ein Winkelwert, kann elektronisch an einen Aktuator übermittelt werden, der sich auch an einem entfernten Ort befinden kann.

Ferner wird auch ein nicht-chirurgisches Instrument mit einer manuellen Steuervorrichtung für eine Betätigung mit der Hand aufgezeigt, wobei bei einer Betätigung der Steuervorrichtung ein Aktuator des chirurgischen Instruments betätigt wird. Es gelten dabei alle zuvor gemachten Erläuterungen bzgl. des chirurgischen Instruments und der Steuervorrichtung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine erste Ausführungsform einer manuellen Steuervorrichtung für eine Betätigung mit der Hand im geöffneten Zustand;
- Fig. 2: die erste Ausführungsform gemäß Fig. 1 im teilgeöffneten Zustand;
- Fig. 3: die erste Ausführungsform gemäß Fig. 1 im geschlossenen Zustand;
- Fig. 4: die Darstellung gemäß Fig. 3, wobei ein anderer teilgeöffneter Zustand mit einer doppelt gestrichelten Linie hinzugefügt wurde;
- Fig. 5: eine erste Ausführungsform eines chirurgischen Instruments mit einer zweite Ausführungsform einer manuellen Steuervorrichtung aus einer ersten Perspektive;
- Fig. 6: die erste Ausführungsform des Instruments gemäß Fig. 5 aus einer zweiten Perspektive;
- Fig. 7: eine dritte Ausführungsform einer manuellen Steuervorrichtung;
- Fig. 8: eine zweite Ausführungsform eines chirurgischen Instruments mit einer Steuervorrichtung gemäß Fig. 7, die mit einem haptischen Eingabegerät gekoppelt ist;
- Fig. 9: die dritte Ausführungsform der Steuervorrichtung gemäß Fig. 7 in einer ersten Seitenansicht;
- Fig. 10: die dritte Ausführungsform der Steuervorrichtung gemäß Fig. 7 in einer zweiten Seitenansicht;
- Fig. 11: die Darstellung gemäß Fig. 9, wobei zusätzlich eine Hand eingezeichnet ist;
- Fig. 12: die Darstellung gemäß Fig. 1, wobei zusätzlich eine Hand eingezeichnet ist,
- Fig. 13: eine vierte Ausführungsform einer manuellen Steuervorrichtung;
- Fig. 14: eine fünfte Ausführungsform einer manuellen Steuervorrichtung,
- Fig. 15: die erste Ausführungsform einer manuellen Steuervorrichtung mit einem pistolenartigen Griff in einer perspektivischen Ansicht,
- Fig. 16: die erste Ausführungsform einer manuellen Steuervorrichtung mit einem pistolenartigen Griff in einer Draufsicht,
- Fig. 17: die zweite Ausführungsform einer manuellen Steuervorrichtung mit einem kugelartigen Griff in einer perspektivischen Ansicht,
- Fig. 18: die zweite Ausführungsform einer manuellen Steuervorrichtung mit einem kugelartigen Griff in einer Draufsicht,
- Fig. 19: die dritte Ausführungsform einer manuellen Steuervorrichtung mit einem stummelartigen Griff in einer ersten perspektivischen Ansicht,
- Fig. 20: die dritte Ausführungsform einer manuellen Steuervorrichtung mit einem stummelartigen Griff in einer zweiten perspektivischen Ansicht, und
- Fig. 21: die dritte Ausführungsform einer manuellen Steuervorrichtung mit einem stummelartigen Griff in einer Draufsicht.

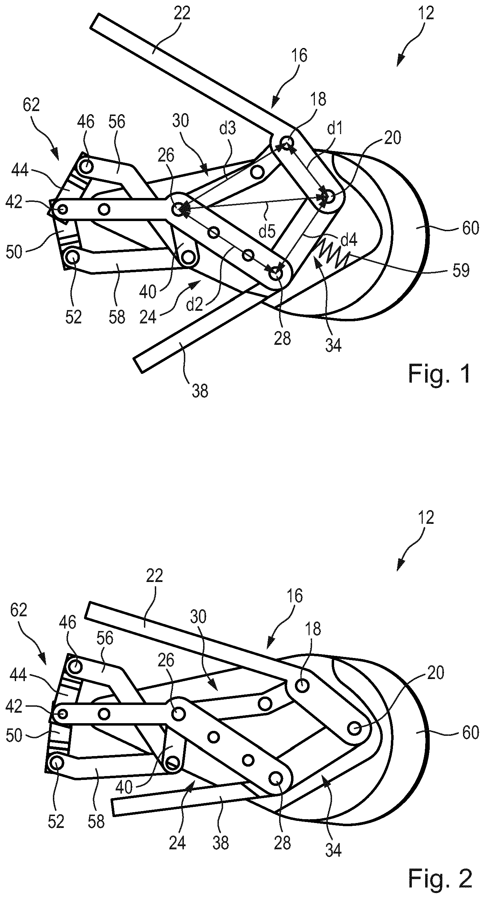

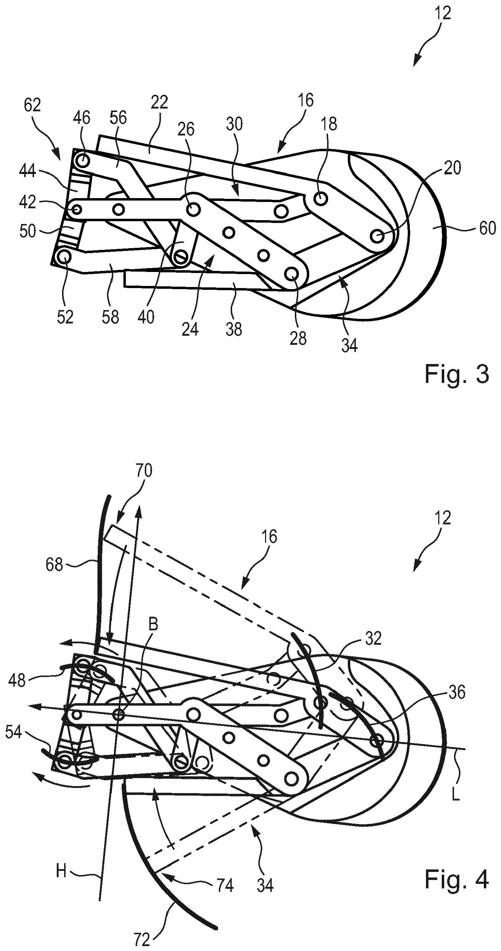

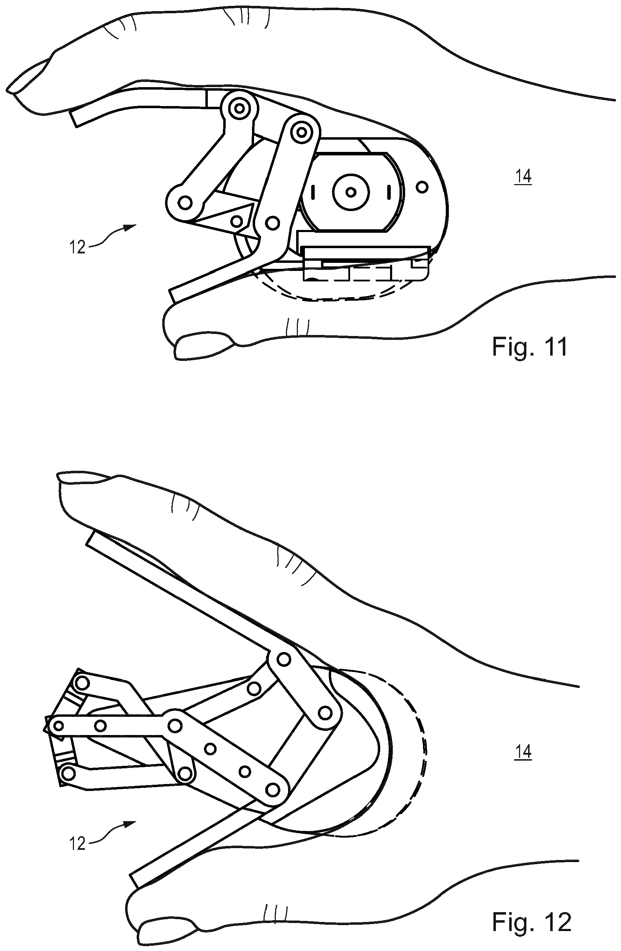

Fig. 1 zeigt eine manuelle Steuervorrichtung 12 für eine Betätigung mit einer Hand 14 (siehe Fig. 12). Die Steuervorrichtung 12 kann zwischen einer geschlossenen Position (siehe Fig. 3) und einer geöffneten Position (siehe Fig. 1) verlagert werden.

Die Steuervorrichtung 12 weist einen Fingerhebel 16 mit einem ersten Anlenkpunkt 18 und einem zweiten Anlenkpunkt 20 auf. Der Fingerhebel 16 weist ferner einen Fingerauflageabschnitt, der ausgehend vom ersten Anlenkpunkt 18 als Fortsatz einer ersten Erstreckung des Fingerhebels 16 zwischen dem zweiten Anlenkpunkt 20 und dem ersten Anlenkpunkt 18 ausgebildet ist. Die Steuervorrichtung 12 weist außerdem ein Zentralelement 24 mit einem dritten Anlenkpunkt 26 und einem vierten Anlenkpunkt 28.

Die manuelle Steuervorrichtung 12 weist außerdem einen Verbindungshebel 30 auf, der am ersten Anlenkpunkt 18 und am dritten Anlenkpunkt 26 verschwenkbar angeordnet ist, so dass der Verbindungshebel 30 relativ zum Zentralelement 24 und relativ zum Fingerhebel 16 verschwenkbar ist und dass der erste Anlenkpunkt 18 entlang einer ersten gekrümmten Bahn 32 (siehe Fig. 4) um den dritten Anlenkpunkt 26 herum verlagerbar ist.

Die Steuervorrichtung 12 weist zudem einen Daumenhebel 34 auf, der am zweiten Anlenkpunkt 20 und am vierten Anlenkpunkt 28 verschwenkbar angeordnet ist, so dass der Daumenhebel 34 relativ zum Zentralelement 24 und relativ zum Fingerhebel 16 verschwenkbar ist und dass der zweite Anlenkpunkt 20 entlang einer zweiten gekrümmten Bahn 36 um den vierten Anlenkpunkt 28 herum geführt verlagerbar ist.

Der Daumenhebel 34 weist einen Daumenauflageabschnitt 38 auf, der ausgehend vom vierten Anlenkpunkt 28 als Fortsatz einer zweiten Erstreckung des Daumenhebels 34 zwischen dem zweiten Anlenkpunkt 20 und dem vierten Anlenkpunkt 28 ausgebildet ist.

Der Verbindungshebel 30 weist einen Betätigungsabschnitt 40 auf, der ausgehend vom dritten Anlenkpunkt 26 als Fortsatz einer dritten Erstreckung des Verbindungshebels 30 zwischen dem ersten Anlenkpunkt 18 und dem dritten Anlenkpunkt 26 ausgebildet ist.

Das Zentralelement 24 weist einen fünften Anlenkpunkt 42 auf. Die Steuervorrichtung 12 weist zudem ein erstes Steuerelement 44 auf, das einen sechsten Anlenkpunkt 46 aufweist und am fünften Anlenkpunkt 42 und am sechsten Anlenkpunkt 46 verschwenkbar angeordnet ist. Das erste Steuerelement 44 ist mit dem Verbindungshebel 30 gekoppelt, so dass das erste Steuerelement 44 relativ zum Zentralelement 24 verschwenkbar ist und dass der sechste Anlenkpunkt 42 entlang einer dritten gekrümmten Bahn 48 (siehe Fig. 4) um den fünften Anlenkpunkt 42 herum geführt verlagerbar ist.

Die Steuervorrichtung 12 weist außerdem ein zweites Steuerelement 50 auf. Dieses zweite Steuerelement 50 ist hier um den fünften Anlenkpunkt 42 verschwenkbar angeordnet, kann aber auch an einem vom fünften Anlenkpunkt 42 verschiedenen siebten Anlenkpunkt (nicht gezeigt) verschwenkbar angeordnet sein. Das zweite Steuerelement 50 weist einen achten Anlenkpunkt 52 auf und ist auch am achten Anlenkpunkt 52 verschwenkbar angeordnet. Das zweite Steuerelement 50 ist mit dem Verbindungshebel 30 gekoppelt, so dass das zweite Steuerelement 50 relativ zum Zentralelement 24 verschwenkbar ist und dass der achte Anlenkpunkt 52 entlang einer vierten gekrümmten Bahn 54 (siehe Fig. 4) um den fünften Anlenkpunkt 42 herum geführt verlagerbar ist.

Der Verbindungshebel 30 und das erste Steuerelement 44 sind mit einer ersten gelenkigen Verbindung gekoppelt, hier mit einem ersten Zwischenhebel 56. Der Verbindungshebel 30 und das zweite Steuerelement 50 sind mit einer zweiten gelenkigen Verbindung gekoppelt, hier mit einem zweiten Zwischenhebel 58.

Der erste Anlenkpunkt 18 und der zweite Anlenkpunkt 20 sind um einen ersten Abstand d1 beabstandet. Der dritte Anlenkpunkt 26 und der vierte Anlenkpunkt 28 sind um einen zweiten Abstand d2 beabstandet. Der erste Abstand d1 beträgt hier ungefähr 55% des zweiten Abstands d2.

Der erste Anlenkpunkt 18 ist vom dritten Anlenkpunkt 26 um einen dritten Abstand d3 beabstandet. Der zweite Anlenkpunkt 20 ist vom vierten Anlenkpunkt 28 um einen vierten Abstand d4 beabstandet. Der vierte Abstand beträgt hier ungefähr 75% des dritten Abstands. Zudem beträgt der erste Abstand ungefähr 70% des vierten Abstands.

Es ist in der Fig. 1 zudem ein optionales federndes Element 59 dargestellt, mit dem die Steuervorrichtung 12 in Richtung einer geöffneten Position vorgespannt werden kann. Dies erleichtert das Öffnen der Steuervorrichtung 12. Das federnde Element 59 kann durch elastische Stoffe gebildet sein und ist hier insbesondere als Schraubenfeder ausgeführt. Es sind aber auch andere Ausgestaltungen, insbesondere als Spiralfeder und als Blattfeder. Das federnde Element 59 greift hier am Daumenhebel 34 an, kann bei entsprechender Positionierung aber auch an jedem anderen Hebel angreifen.

Die Steuervorrichtung 12 ist derart ausgebildet, dass sich bei einem Öffnen der Steuervorrichtung 12 ein fünfter Abstand d5 zwischen dem zweiten Anlenkpunkt 20 und dem dritten Anlenkpunkt 26 verringert. Dies wird in den nachfolgenden Figuren verdeutlicht. In entsprechender Weise vergrößert sich der Abstand d5 bei einem Schließen der Steuervorrichtung 12.

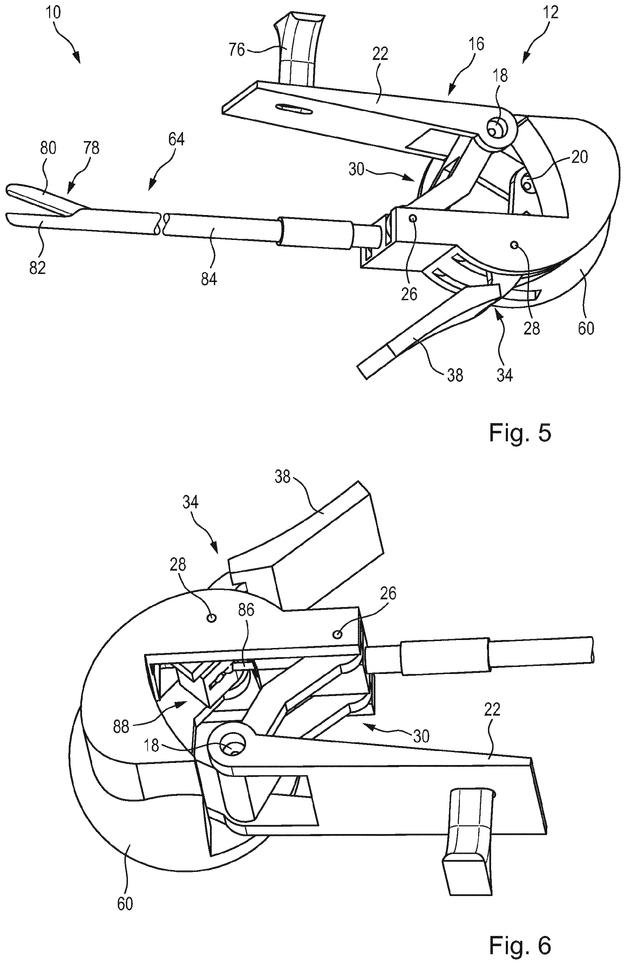

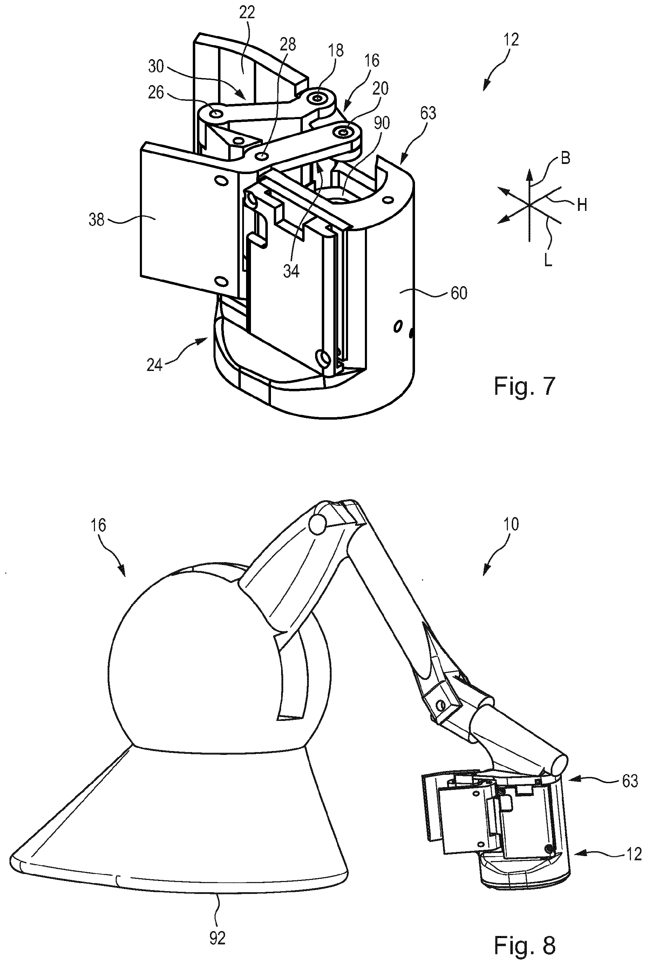

Das Zentralelement 24 weist eine Handballenauflage 60 auf, auf der eine Hand ergonomisch über eine längere Zeit, insbesondere über mehrere Stunden, aufliegen kann, insbesondere ermüdungsfrei aufliegen kann. Bei der hier gezeigten Ausführungsform weist das Zentralelement 24 außerdem einen Adapter 62 für eine Kopplung mit einem Arbeitsabschnitt 64 (siehe Fig. 5) oder, wenn ein anderer Adapter 63 verwendet wird, mit einem haptischen Eingabegerät 66 (siehe Fig. 8) auf. Der Adapter 62 für eine Kopplung mit einem Arbeitsabschnitt 64 weist hier das erste Steuerelement 44 und das zweite Steuerelement 50 auf.

Die in Fig. 1 gezeigte Position der Steuervorrichtung 12 wird hier als geöffneter Zustand bezeichnet. Es sei darauf hingewiesen, dass die Steuervorrichtung 12 auch weiter geöffnet werden kann, als es hier gezeigt ist.

Fig. 2 zeigt die Steuervorrichtung 12 gemäß Fig. 1 in einem teilgeöffneten Zustand. Es ist zu erkennen, dass sich der fünfte Abstand d5 vergrößert hat. Zudem hat sich die Position des Adapters 62, konkret die Ausrichtung des ersten Steuerelements 44 und des zweiten Steuerelements 50 verändert. Ist am fünften Anlenkpunkt 42 ein feststehender Teil des Arbeitsschnitts 64 angeordnet und am ersten Steuerelement 44 und/oder am zweiten Steuerelement 50 zumindest ein beweglicher Teil des Arbeitsabschnitts 64 angekoppelt, so kann die Verlagerung des ersten und/oder zweiten Steuerelements 44, 50 für eine Betätigung des Arbeitsabschnitts verwendet werden.

Fig. 3 zeigt schließlich die erste Ausführungsform gemäß Fig. 1 in einem geschlossenen Zustand.

Fig. 4 zeigt in einer Überlagerung die Darstellung gemäß Fig. 3 mit einer durchgezogenen Linie und die Darstellung gemäß Fig. 1 mit einer doppeltgestrichelten Linie. Bei dieser Darstellung können die Bewegungen der einzelnen Teile gut nachvollzogen werden. Es sind zudem die zuvor erläuterten Bewegungsbahnen eingezeichnet.

Als Orientierungshilfe sind hier zudem eine Längsrichtung L der Steuervorrichtung 12 und eine dazu senkrecht verlaufende Höhenrichtung H dargestellt. Eine Richtung, die sowohl senkrecht zur Längsrichtung L als auch senkrecht zur Höhenrichtung H verläuft, ist als Breitenrichtung B eingezeichnet und steht hier senkrecht zur Zeichenebene.

Zusätzlich ist eine Fingerbewegungsbahn 68 eingezeichnet, die die Bewegung des ersten distalen Endes 70 des Fingerhebels 16 beschreibt. Außerdem ist eine Daumenbewegungsbahn 72 eingezeichnet, die die Bewegung eines zweiten distalen Endes 74 des Daumenhebels 34 beschreibt. Bei dieser Darstellung wurden nicht alle bisher eingeführten Bezugszeichen wiederholt, um die Übersichtlichkeit der Darstellung zu gewährleisten. Hier und im Folgenden gelten aber alle eingeführten Bezugszeichen für identische oder funktional gleiche Elemente fort.

Fig. 5 zeigt eine erste Ausführungsform eines chirurgischen Instruments 10 mit einer zweiten Ausführungsform einer Steuervorrichtung 12. Die zuvor gemachten Erläuterungen im Zusammenhang mit dem Zentralelement, dem Fingerhebel, dem Daumenhebel und dem Verbindungshebel gelten auch hier. Um die Aufwärtsbewegung beim Öffnen der Steuervorrichtung 12 zu erleichtern ist hier am Fingerhebel 16, genauer an dem Fingerauflageabschnitt 22 ein Fingerhalter 76, auch als Fingerbolzen bezeichnet, angeordnet, wobei der Fingerhalter 76 bei bestimmen Ausführungsformen (nicht gezeigt) auch Fingerschlaufen aufweisen kann. Der Arbeitsabschnitt 64 weist einen Aktuator 78 mit einem beweglichen Maulteil 80 und einem feststehenden Maulteil 82 auf. Ferner hat der Arbeitsabschnitt 64 einen feststehenden Teil 84 und einen verlagerbaren Teil 86 (siehe Fig. 6), der mit einem Adapter 88 (siehe Fig. 6) gekoppelt ist. Adapter 88 ist hier mit dem Daumenhebel 34 lösbar fixiert.

In Fig. 6, die die Darstellung gemäß Fig. 5 aus einer anderen Perspektive zeigt, ist zu erkennen, dass eine Bewegung des Daumenhebels einen positiven oder negativen Schub auf das bewegliche Element 86, hier eine Schubstange oder ein Bowdenzug, ausüben kann. Die Ankopplung über das bewegliche Teil 86 mit dem beweglichen Maulstück 80 erfolgt dergestalt, z.B. über ein Zahnrad mit einer Zahnstange, das ein Öffnen der Steuervorrichtung 10 den Aktuator 78 öffnet und ein Schließen der Steuervorrichtung 10 den Aktuator 78 schließt.

Fig. 7 zeigt eine dritte Ausführungsform einer Steuervorrichtung 12. Es gelten alle vorherigen Erläuterungen zum Zentralelement, dem Daumenhebel, dem Fingerhebel und dem Verbindungshebel fort. Die dritte Ausführungsform weist einen Adapter 63 für eine Kopplung mit einem haptischen Eingabegerät 66 des chirurgischen Instruments 10 auf. Zudem ist, wie nachfolgend noch verdeutlicht wird, auch ein Motor 90 in der Steuervorrichtung 12 angeordnet, der hier mit dem Daumenhebel 34 gekoppelt ist. Durch den Einsatz des Motors 90 kann eine Betätigung durch den Benutzer unterstützt werden, ein bestimmter Öffnungsgrad der Steuervorrichtung 10 gehalten werden oder eine haptische Rückmeldung (Kraftrückmeldung, Force Feedback) ermöglicht werden. Zudem kann der Motor 90 auch so betrieben werden, dass er kontinuierlich versucht, die Steuervorrichtung 12 zu öffnen bzw. die Steuervorrichtung 12 mit einer Kraft in Richtung des geöffneten Zustands zu beaufschlagen.

Fig. 8 zeigt eine zweite Ausführungsform eines chirurgischen Instruments mit einem Arbeitsabschnitt 91, der hier aufgrund von Steuersignalen des haptischen Eingabegeräts 66 betätigt wird.

Es ist hier zu erkennen, dass die Breite der Steuervorrichtung 12, also die Erstreckung entlang der Breitenrichtung B, mindestens 50% der Breite einer menschlichen Hand beträgt, bevorzugt mindestens 75%, besonders bevorzugt mindestens 85% und insbesondere 95%.

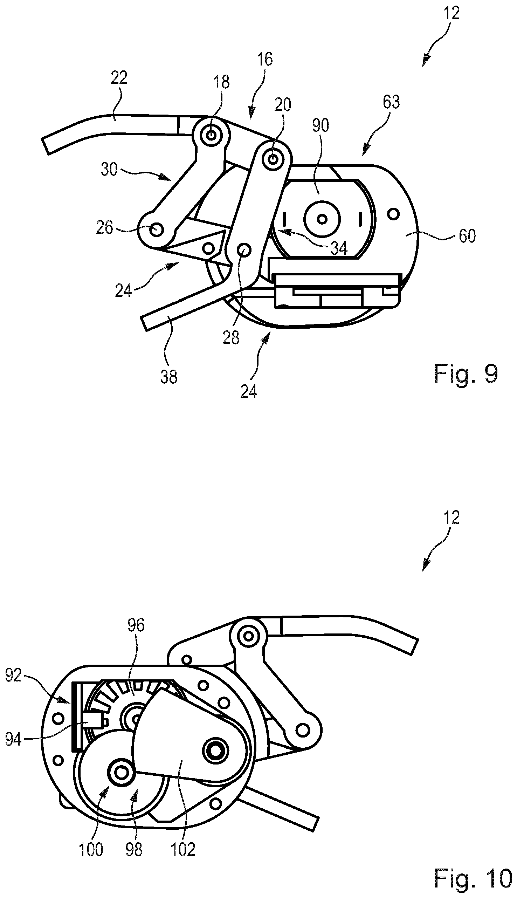

Fig. 9 zeigt die Darstellung gemäß Fig. 7 in einer ersten Seitenansicht. Fig. 10 zeigt die Darstellung gemäß Fig. 7 in einer zweiten Seitenansicht. Es ist hier eine zweite Messeinrichtung 92 zu erkennen, die dafür ausgebildet ist, einen Öffnungsgrad der Steuervorrichtung 12 zu ermitteln, insbesondere einen Öffnungswinkel der Steuervorrichtung. Hierfür weist die Messeinrichtung 92 einen Sensor 94 und einen Signalgeber 96 auf, der hier mit dem Daumenhebel 34 gekoppelt ist, insbesondere über ein Getriebe.

Der Signalgeber 96 kann bei anderen Ausgestaltungen auch mit dem Verbindungshebel 30 oder mit dem Fingerhebel 16 gekoppelt sein. Ferner ist ein Übertragungsmechanismus gezeigt, der ein Drehmoment des Motors 90 auf ein kleines Rad 100 überträgt, insbesondere eine Zahnrad, das über einen Übertragungshebel 102 mit dem Daumenhebel 34 gekoppelt ist. Auf diese Weise kann der Motor 90 mit starker Untersetzung mit dem Daumenhebel 34 gekoppelt werden.

Fig. 11 zeigt die dritte Ausführungsform der Steuervorrichtung 12 mit einer aufgelegten Hand 14.

Fig. 12 zeigt die erste Ausführungsform der Steuervorrichtung 12 mit einer aufgelegten Hand 14.

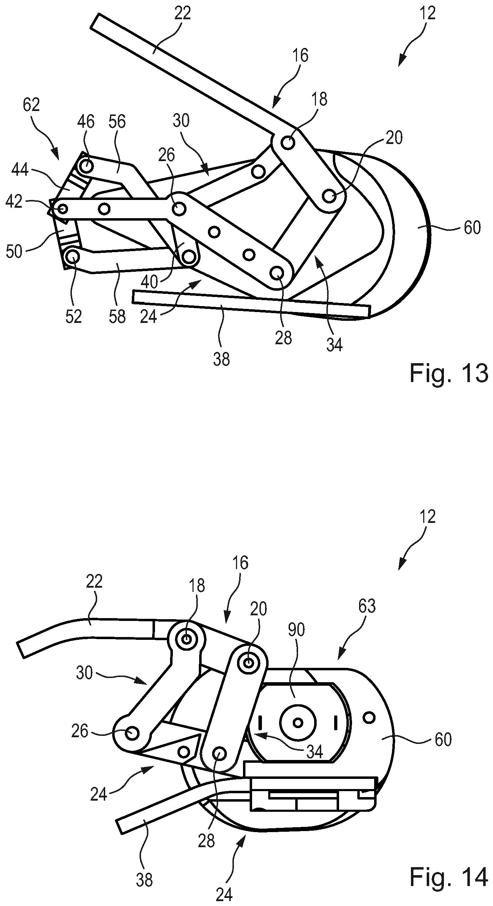

Fig. 13 zeigt eine vierte Ausführungsform einer manuellen Steuervorrichtung 12 mit einem Daumenauflageabschnitt 38, der nicht als Fortsatz des Daumenhebels ausgebildet ist. Der Daumenauflageabschnitt 38 ist hier relativ zum Zentralelement 24 fixiert. Beim Öffnen und Schließen der Steuervorrichtung 12 verlagert der Benutzer nur seine Finger. Es gibt auch bevorzugte Ausgestaltungen, bei denen ein Daumenauflageabschnitt gar nicht vorhanden ist.

Fig. 14 zeigt eine fünfte Ausführungsform einer manuellen Steuervorrichtung 12, bei der der Daumenauflageabschnitt 38 ebenfalls relativ zum Zentralelement 24 fixiert ist.

Die Ausgestaltung der Handballenauflage 60 bzw. des Griffstücks des Griffs des chirurgischen Instruments 10 ist wichtig für ein ergonomisches Greifen. Im Folgenden werden drei der besonders bevorzugten Ausgestaltungen beschrieben, die als besonders ergonomisch angesehen werden.

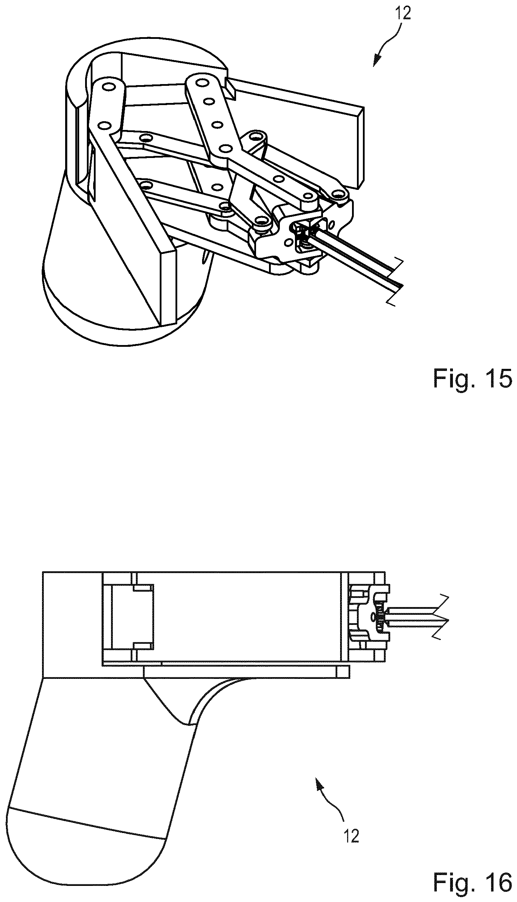

Figs. 15 und 16 zeigen die erste Ausführungsform einer manuellen Steuervorrichtung 12 mit einem pistolenartigen Griff in einer perspektivischen Ansicht bzw. in einer Draufsicht. Es gelten alle Bezugszeichen weiter, wie sie bereits eingeführt wurden, und werden hier nicht wiederholt.

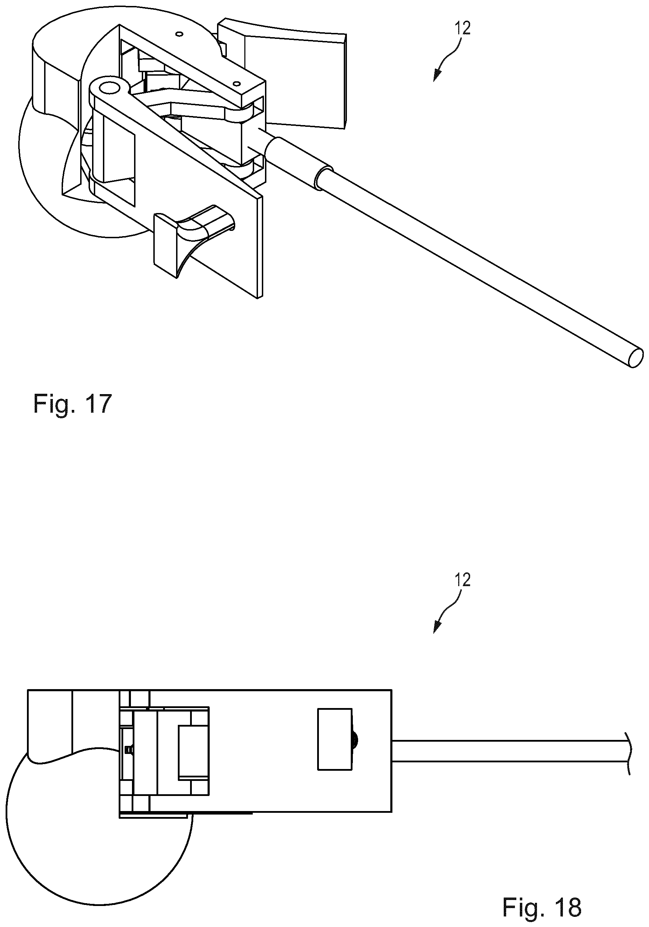

Figs. 17 und 18 zeigen die zweite Ausführungsform einer manuellen Steuervorrichtung 12 mit einem kugelartigen Griff in einer perspektivischen Ansicht bzw. in einer Draufsicht. Es gelten alle Bezugszeichen weiter, wie sie bereits eingeführt wurden, und werden hier nicht wiederholt.

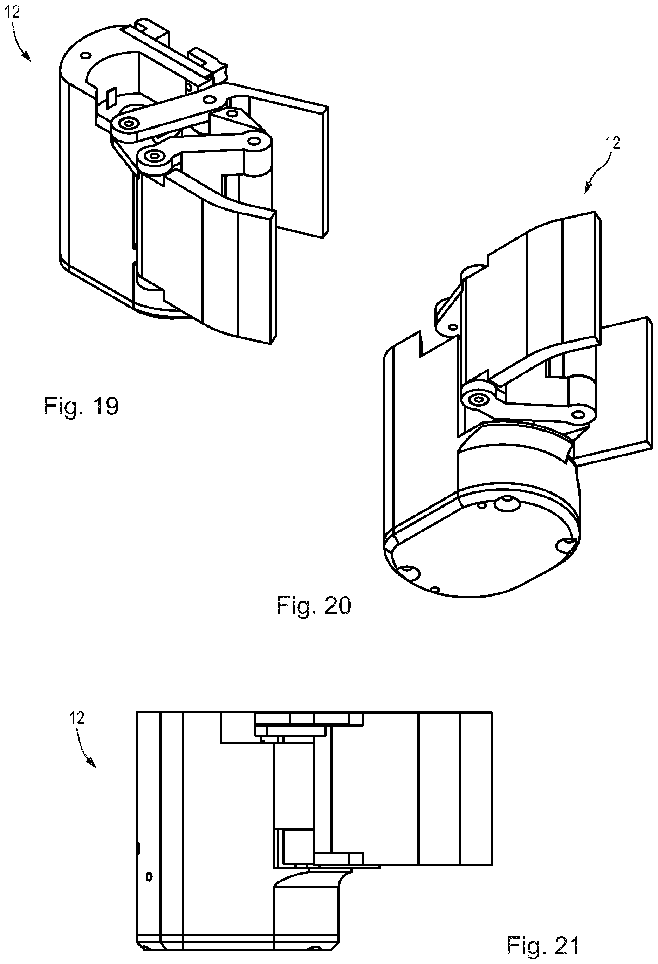

Figs. 19, 20 und 21 zeigen die erste Ausführungsform einer manuellen Steuervorrichtung 12 mit einem stummelartigen Griff in einer ersten und zweiten perspektivischen Ansicht bzw. in einer Draufsicht. Es gelten alle Bezugszeichen weiter, wie sie bereits eingeführt wurden, und werden hier nicht wiederholt.

## Patentansprüche

1. Chirurgisches Instrument (10) mit einer manuellen Steuervorrichtung (12) für eine Betätigung mit einer Hand (14), wobei die Steuervorrichtung (12) zwischen einer geschlossenen Position und einer geöffneten Position verlagert werden kann und die Steuervorrichtung (12) aufweist:
- einen Fingerhebel (16) mit einem ersten Anlenkpunkt (18) und einem zweiten Anlenkpunkt (20), wobei der Fingerhebel einen Fingerauflageabschnitt (22) aufweist, der ausgehend vom ersten Anlenkpunkt (18) als Fortsatz einer ersten Erstreckung des Fingerhebels (16) zwischen dem zweiten Anlenkpunkt (20) und dem ersten Anlenkpunkt (18) ausgebildet ist,
- ein Zentralelement (24) mit einem dritten Anlenkpunkt (26) und einem vierten Anlenkpunkt (28),
- einen Verbindungshebel (30), der am ersten Anlenkpunkt (18) und am dritten Anlenkpunkt (26) verschwenkbar angeordnet ist, so dass der Verbindungshebel (30) relativ zum Zentralelement (24) und relativ zum Fingerhebel (16) verschwenkbar ist und dass der erste Anlenkpunkt (18) entlang einer ersten gekrümmten Bahn (32) um den dritten Anlenkpunkt (26) herum durch den Fingerhebel (16) geführt verlagerbar ist bei einer Verlagerung der Steuervorrichtung (12) zwischen der geschlossenen Position und der geöffneten Position,
- einen Daumenhebel (34), der am zweiten Anlenkpunkt (20) und am vierten Anlenkpunkt (28) verschwenkbar angeordnet ist, so dass der Daumenhebel (34) relativ zum Zentralelement (24) und relativ zum Fingerhebel (16) verschwenkbar ist und dass der zweite Anlenkpunkt (20) entlang einer zweiten gekrümmten Bahn (36) um den vierten Anlenkpunkt (28) herum durch den Daumenhebel (34) geführt verlagerbar ist bei der Verlagerung der Steuervorrichtung (12) zwischen der geschlossenen Position und der geöffneten Position.

2. Chirurgisches Instrument nach Anspruch 1, wobei der Daumenhebel (34) einen Daumenauflageabschnitt (38) aufweist, der ausgehend vom vierten Anlenkpunkt (28) als Fortsatz einer zweiten Erstreckung des Daumenhebels (34) zwischen dem zweiten Anlenkpunkt (20) und dem vierten Anlenkpunkt (28) ausgebildet ist.

3. Chirurgisches Instrument nach einem der vorherigen Ansprüche, wobei der Verbindungshebel (30) einen Betätigungsabschnitt (40) aufweist, der ausgehend vom dritten Anlenkpunkt (26) als Fortsatz einer dritten Erstreckung des Verbindungshebels zwischen dem ersten Anlenkpunkt (18) und dem dritten Anlenkpunkt (26) ausgebildet ist.

4. Chirurgisches Instrument nach einem der vorherigen Ansprüche, wobei das Zentralelement (24) einen fünften Anlenkpunkt (42) aufweist und die Steuervorrichtung (12) ein erstes Steuerelement (44) aufweist, das einen sechsten Anlenkpunkt (46) aufweist, am fünften Anlenkpunkt (42) und am sechsten Anlenkpunkt (46) verschwenkbar angeordnet ist und mit dem Verbindungshebel (30) gekoppelt ist, so dass das erste Steuerelement (44) relativ zum Zentralelement (24) verschwenkbar ist und dass der sechste Anlenkpunkt (42) entlang einer dritten gekrümmten Bahn (48) um den fünften Anlenkpunkt (42) herum geführt verlagerbar ist.

5. Chirurgisches Instrument nach einem der vorherigen Ansprüche, wobei das Zentralelement einen siebten Anlenkpunkt aufweist und die Steuervorrichtung (12) ein zweites Steuerelement (50) aufweist, das einen achten Anlenkpunkt (52) aufweist, am siebten Anlenkpunkt und am achten Anlenkpunkt (52) verschwenkbar angeordnet ist und mit dem Verbindungshebel (30) gekoppelt ist, so dass das zweite Steuerelement (50) relativ zum Zentralelement (24) verschwenkbar ist und dass der achte Anlenkpunkt (52) entlang einer vierten gekrümmten Bahn (54) um den siebten Anlenkpunkt herum geführt verlagerbar ist.

6. Chirurgisches Instrument nach Anspruch 4, wobei der Verbindungshebel (30) und das erste Steuerelement (44) mit einer ersten gelenkigen Verbindung gekoppelt sind, insbesondere mit einem ersten Zwischenhebel (56).

7. Chirurgisches Instrument nach Anspruch 5, wobei der Verbindungshebel (30) und das zweite Steuerelement (50) mit einer zweiten gelenkigen Verbindung gekoppelt sind, insbesondere mit einem zweiten Zwischenhebel (58).

8. Chirurgisches Instrument nach einem der vorherigen Ansprüche, wobei der erste Anlenkpunkt (18) um einen ersten Abstand (d1) vom zweiten Anlenkpunkt (20) beabstandet ist, der dritte Anlenkpunkt (26) um einen zweiten Abstand (d2) vom vierten Anlenkpunkt (28) beabstandet ist und der erste Abstand (d1) zwischen 25% und 125% des zweiten Abstands beträgt, bevorzugt zwischen 35% und 100%, besonders bevorzugt zwischen 45% und 75% und insbesondere zwischen 50% und 60%.

9. Chirurgisches Instrument nach einem der vorherigen Ansprüche, wobei der erste Anlenkpunkt (18) um einen dritten Abstand (d3) vom dritten Anlenkpunkt (26) beabstandet ist, der zweite Anlenkpunkt (20) um einen vierten Abstand vom vierten Anlenkpunkt (28) beabstandet ist und der vierte Abstand (d4) zwischen 35% und 150% des dritten Abstands (d3) beträgt, bevorzugt zwischen 45% und 125%, besonders bevorzugt zwischen 55% und 100% und insbesondere zwischen 65% und 85%.

10. Chirurgisches Instrument nach einem der vorherigen Ansprüche, wobei der erste Anlenkpunkt (18) um einen ersten Abstand (d1) vom zweiten Anlenkpunkt (20) beabstandet ist, der zweite Anlenkpunkt (20) um einen vierten Abstand (d4) vom vierten Anlenkpunkt (28) beabstandet ist und der erste Abstand (d1) zwischen 40% und 150% des dritten Abstands (d3) beträgt, bevorzugt zwischen 45% und 125%, besonders bevorzugt zwischen 50% und 100% und insbesondere zwischen 60% und 80%.

11. Chirurgisches Instrument nach einem der vorherigen Ansprüche, ferner mit einer Schubstange (86), die gelenkig mit mindestens einem Element ausgewählt aus der Gruppe von Fingerhebel (16), Daumenhebel (34) und Verbindungshebel (30) gekoppelt ist.

12. Chirurgisches Instrument nach einem der vorherigen Ansprüche, wobei die Steuervorrichtung (10) derart ausgebildet ist, dass sich bei einem Öffnen der Steuervorrichtung (10) ein fünfter Abstand (d5) zwischen dem zweiten Anlenkpunkt (20) und dem dritten Anlenkpunkt (26) verringert.

13. Chirurgisches Instrument nach einem der vorherigen Ansprüche, wobei das Zentralelement (24) eine Handballenauflage (60) und/oder einen Adapter (62, 63, 76) für eine Kopplung mit einem Arbeitsabschnitt (64, 91) des chirurgischen Instruments (10) oder mit einem haptischen Eingabegerät (66) des chirurgischen Instruments (10) aufweist.

14. Chirurgisches Instrument nach einem der vorherigen Ansprüche, ferner mit einem Motor (90), der mit mindestens einem Element ausgewählt aus der Gruppe von Fingerhebel (16), Daumenhebel (34) und Verbindungshebel (30) zusammenwirkt, um eine haptische Rückmeldung und/oder eine motorische Unterstützung zu geben.

15. Chirurgisches Instrument nach einem der vorherigen Ansprüche, ferner mit einer Messeinrichtung (92), die dafür ausgebildet ist, einen Öffnungsgrad der Steuervorrichtung (12) zu ermitteln, insbesondere einen Öffnungswinkel der Steuervorrichtung.

## Claims

1. A surgical instrument (10) having a manual control device (12) for operation by hand (14), the control device (12) being displaceable between a closed position and an open position, the control device (12) comprising:
- a finger lever (16) having a first pivot point (18) and a second pivot point (20), the finger lever having a finger support section (22) which, starting from the first pivot point (18), is designed as an extension of a first extension of the finger lever (16) between the second pivot point (20) and the first pivot point (18),
- a central element (24) having a third pivot point (26) and a fourth pivot point (28),
- a connecting lever (30) pivotally disposed at the first pivot point (18) and the third pivot point (26) such that the connecting lever (30) is pivotable relative to the central element (24) and relative to the finger lever (16), and that the first pivot point (18) is displaceable along a first curved path (32) around the third pivot point (26) guided by the finger lever (16) upon displacement of the control device (12) between the closed position and the open position,
- a thumb lever (34) which is pivotably arranged at the second pivot point (20) and at the fourth pivot point (28), so that the thumb lever (34) is pivotable relative to the central element (24) and relative to the finger lever (16), and that the second pivot point (20) is displaceable along a second curved path (36) around the fourth pivot point (28) guided by the thumb lever (34) upon displacement of the control device (12) between the closed position and the open position.

2. The surgical instrument according to claim 1, wherein the thumb lever (34) has a thumb rest portion (38) which, starting from the fourth pivot point (28), is formed as an extension of a second extension of the thumb lever (34) between the second pivot point (20) and the fourth pivot point (28).

3. The surgical instrument according to one of the preceding claims, the connecting lever (30) having an actuating portion (40) formed as an extension of a third extension of the connecting lever between the first pivot point (18) and the third pivot point (26) starting from the third pivot point (26).

4. The surgical instrument according to one of the preceding claims, wherein the central element (24) has a fifth pivot point (42) and the control device (12) has a first control member (44) having a sixth pivot point (46), is pivotally arranged at the fifth pivot point (42) and at the sixth pivot point (46) and is coupled to the connecting lever (30) so that the first control element (44) is pivotable relative to the central element (24) and that the sixth pivot point (42) is displaceable along a third curved path (48) guided around the fifth pivot point (42).

5. The surgical instrument according to one of the preceding claims, wherein the central element has a seventh pivot point and the control device (12) comprises a second control member (50) having an eighth pivot point (52), is pivotally arranged at the seventh pivot point and at the eighth pivot point (52) and is coupled to the connecting lever (30) so that the second control element (50) is pivotable relative to the central element (24) and that the eighth pivot point (52) is displaceable along a fourth curved path (54) guided around the seventh pivot point.

6. The surgical instrument according to claim 4, wherein the connecting lever (30) and the first control element (44) are coupled to a first articulated connection, in particular to a first intermediate lever (56).

7. The surgical instrument according to claim 5, the connecting lever (30) and the second control element (50) being coupled to a second articulated connection, in particular to a second intermediate lever (58).

8. The surgical instrument according to one of the preceding claims, wherein the first pivot point (18) is spaced from the second pivot point (20) by a first distance (d1), the third pivot point (26) is spaced from the fourth pivot point (28) by a second distance (d2), and the first distance (d1) is between 25% and 125% of the second distance, preferably between 35% and 100%, particularly preferably between 45% and 75% and in particular between 50% and 60%.

9. The surgical instrument according to one of the preceding claims, wherein the first pivot point (18) is spaced from the third pivot point (26) by a third distance (d3), the second pivot point (20) is spaced from the fourth pivot point (28) by a fourth distance (d4) and the fourth distance (d4) is between 35% and 150% of the third distance (d3), preferably between 45% and 125%, particularly preferably between 55% and 100% and in particular between 65% and 85%.

10. The surgical instrument according to one of the preceding claims, wherein the first pivot point (18) is spaced from the second pivot point (20) by a first distance (d1), the second pivot point (20) is spaced from the fourth pivot point (28) by a fourth distance (d4), and the first distance (d1) is between 40% and 150% of the third distance (d3), preferably between 45% and 125%, particularly preferably between 50% and 100% and particularly between 60% and 80%.

11. The surgical instrument according to one of the preceding claims, further comprising a push rod (86) hingedly coupled to at least one member selected from the group consisting of finger lever (16), thumb lever (34) and connecting lever (30).

12. The surgical instrument according to one of the preceding claims, wherein the control device (10) is arranged such that, when the control device (10) is opened, a fifth distance (d5) between the second pivot point (20) and the third pivot point (26) decreases.

13. The surgical instrument according to one of the preceding claims, wherein the central element (24) comprises a palm rest (60) and/or an adapter (62, 63, 76) for coupling with a working portion (64, 91) of the surgical instrument (10) or with a haptic input device (66) of the surgical instrument (10).

14. The surgical instrument according to one of the preceding claims, further comprising a motor (90) co-operating with at least one element selected from the group of finger lever (16), thumb lever (34) and connecting lever (30) to provide haptic feedback and/or motor support.

15. The surgical instrument according to one of the preceding claims, further comprising a measuring device (92) adapted to determine a degree of opening of the control device (12), in particular an angle of opening of the control device.

## Revendications

1. Instrument chirurgical (10) comportant un dispositif de contrôle manuelle (12) destiné à fonctionner avec une main (14), le dispositif de contrôle (12) pouvant être déplacé entre une position fermée et une position ouverte, le dispositif de contrôle (12) comprenant :
- un levier à doigt (16) ayant un premier point de pivotement (18) et un second point de pivotement (20), le levier à doigt ayant une section de support de doigt (22) qui, à partir du premier point de pivotement (18), est conçue comme une extension d'une première extension du levier à doigt (16) entre le second point de pivotement (20) et le premier point de pivotement (18),
- un élément central (24) ayant un troisième point de pivotement (26) et un quatrième point de pivotement (28),
- un levier à liaison (30) disposé de manière pivotante au dudit premier point de pivotement (18) et dudit troisième point de pivotement (26) de telle sorte que ledit levier à liaison (30) peut pivoter par rapport audit élément central (24) et par rapport audit levier à doigt (16), et que ledit premier point de pivotement (18) peut être déplacé le long d'une premier trajectoire courbe (32) autour dudit troisième point de pivotement (26) guidé par ledit levier à doigt (16), lorsque ledit dispositif de contrôle (12) est déplacé entre ladite position fermée et ladite position ouverte,
- un levier à pouce (34) qui est disposé de manière pivotante au deuxième point de pivotement (20) et au quatrième point de pivotement (28), de sorte que le levier à pouce (34) peut pivoter par rapport à l'élément central (24) et par rapport au levier à doigt (16), et que le deuxième point de pivotement (20) peut être déplacé le long d'une deuxième trajectoire courbe (36) autour dudit quatrième point de pivotement (28) guidé par le levier à pouce (34), lorsque le dispositif de contrôle (12) est déplacé entre la position fermée et la position ouverte.

2. Instrument chirurgical selon la revendication 1, dans lequel le levier à pouce (34) présente une partie d'appui de pouce (38) qui, à partir du quatrième point de pivotement (28), est formée comme prolongement d'une deuxième extension du levier à pouce (34) entre le deuxième point de pivotement (20) et le quatrième point de pivotement (28).

3. Instrument chirurgical selon l'une des revendications précédentes, le levier à liaison (30) ayant une partie d'actionnement (40) formée comme une extension d'une troisième extension du levier à liaison entre le premier point de pivotement (18) et le troisième point de pivotement (26) partant du troisième point de pivotement (26).

4. Instrument chirurgical selon l'une des revendications précédentes, dans lequel l'élément central (24) présente un cinquième point de pivotement (42) et le dispositif de contrôle (12) présente un premier élément de commande (44) présentant un sixième point de pivotement (46), est disposé de manière pivotante au cinquième point de pivotement (42) et au sixième point de pivotement (46) et est couplé au levier à liaison (30) de sorte que le premier élément de commande (44) peut pivoter par rapport à l'élément central (24) et que le sixième point de pivotement (42) peut être déplacé le long d'une troisième trajectoire courbe (48) qui contourne le cinquième point de pivotement (42).

5. Instrument chirurgical selon l'une des revendications précédentes, dans lequel l'élément central a un septième point de pivotement et le dispositif de contrôle (12) comprend un deuxième élément de commande (50) ayant un huitième point de pivotement (52), est disposé de manière pivotante au septième point de pivotement et au huitième point de pivotement (52) et est couplé au levier à liaison (30) de sorte que le deuxième élément de commande (50) peut pivoter par rapport à l'élément central (24) et que le huitième point de pivotement (52) peut être le long d'une quatrième trajectoire courbe (54) qui contourne le septième point de pivotement.

6. Instrument chirurgical selon la revendication 4, dans lequel le levier à liaison (30) et le premier élément de commande (44) sont couplés à une première liaison articulée, en particulier à un premier levier intermédiaire (56).

7. Instrument chirurgical selon la revendication 5, le levier à liaison (30) et le deuxième élément de commande (50) étant couplés à une deuxième liaison articulée, en particulier à un deuxième levier intermédiaire (58).

8. Instrument chirurgical selon l'une des revendications précédentes, dans lequel le premier point de pivotement (18) est espacé du deuxième point de pivotement (20) d'une première distance (d1), le troisième point de pivotement (26) est espacé du quatrième point de pivotement (28) d'une deuxième distance (d2) et la première distance (d1) est entre 25% et 125% de la deuxième distance, de préférence entre 35% et 100%, surtout de préférence entre 45% et 75%, notamment entre 50% et 60%.

9. Instrument chirurgical selon l'une des revendications précédentes, dans lequel le premier point de pivotement (18) est espacé du troisième point de pivotement (26) d'une troisième distance (d3), le deuxième point de pivotement (20) est espacé du quatrième point de pivotement (28) d'une quatrième distance (d4) et la quatrième distance (d4) est entre 35% et 150% de la troisième distance (d3), de préférence entre 45% et 125%, particulièrement de préférence entre 55% et 100%, notamment entre 65% et 85%.

10. Instrument chirurgical selon l'une des revendications précédentes, dans lequel le premier point de pivotement (18) est espacé du deuxième point de pivotement (20) d'une première distance (d1), le deuxième point de pivotement (20) est espacé du quatrième point de pivotement (28) d'une quatrième distance (d4) et la première distance (d1) est entre 40% et 150% de la troisième distance (d3), de préférence entre 45% et 125%, surtout de préférence entre 50% et 100%, particulièrement entre 60% et 80%.

11. Instrument chirurgical selon l'une des revendications précédentes, comprenant en outre une tige de poussée (86) couplée de manière articulée à au moins un élément choisi dans le groupe constitué par le levier à doigt (16), le levier à pouce (34) et le levier à connexion (30).

12. Instrument chirurgical selon l'une des revendications précédentes, dans lequel le dispositif de contrôle (10) est disposé de telle sorte que, lorsque le dispositif de contrôle (10) est ouvert, une cinquième distance (d5) entre le deuxième point de pivotement (20) et le troisième point de pivotement (26) diminue.

13. Instrument chirurgical selon l'une des revendications précédentes, dans lequel l'élément central (24) comprend un repose-poignet (60) et/ou un adaptateur (62, 63, 76) pour couplage avec une partie de travail (64, 91) de l'instrument chirurgical (10) ou avec un dispositif haptique (66) de l'instrument chirurgical (10).

14. Instrument chirurgical selon l'une des revendications précédentes, comprenant en outre un moteur (90) coopérant avec au moins un élément choisi dans le groupe du levier à doigt (16), du levier à pouce (34) et du levier à connexion (30) pour fournir un feedback haptique et/ou un support moteur.

15. Instrument chirurgical selon l'une des revendications précédentes, comprenant en outre un dispositif de mesure (92) adapté pour déterminer un degré d'ouverture du dispositif de contrôle (12), en particulier un angle d'ouverture du dispositif de contrôle.
